# EUROPÄISCHE PATENTANMELDUNG

(11) **EP 0 793 095 A1**
(43) Veröffentlichungstag der Anmeldung: **03.09.1997**
(21) Anmeldenummer: 97102367.6
(22) Anmeldetag: 14.02.1997
(51) Int. Cl.: G01N 27/327, C12Q 1/00

(54) **Durch Festkörper unterstützte Membran-Biosensoren**

(30) Priorität: 27.02.1996 DE 19607279
(71) Anmelder: BAYER AG, 51368 Leverkusen (DE)
(72) Erfinder: Galla, Hans-Joachim, Dr., 48159 Münster (DE); Steinem, Claudia, 48153 Münster (DE); Reihs, Karsten, Dr., 50678 Köln (DE)

(57) **Zusammenfassung**

Die neuen Membran-Biosensoren bestehen aus einem Festkörper A als Träger, einer Lipiddoppelschicht B als Membran, zwischen A und B eingebauten Spacern C und einem in die Lipiddoppelschicht eingelagerten Rezeptor D. Die neuen Membran-Biosensoren sind insbesondere durch ihre Spacer C gekennzeichnet, die aus einem Molekül Ethanolamin, das eine Esterbindung zur Phosphatidgruppe der Lipiddoppelschicht B ausbildet, einem Oligopeptid in Helix- oder Faltblattstruktur aus 4-20 C₂-C₁₀-α-Aminosäuren und einer Ankergruppe, die eine chemische oder physikochemische Bindung mit dem Träger A eingeht, besteht.

## Beschreibung

Die Erfindung betrifft neue Membran-Biosensoren, die aus einem Festkörper als Träger, einer Lipiddoppelschicht als Membran, welche über einen Spacer an den Festkörper gebunden ist, und einem in die Lipiddoppelschicht eingelagerten Rezeptor, der den Festkörper nicht berührt, bestehen. Die neuen Membran-Biosensoren sind vor allem durch ihre speziellen Spacer, die ein Oligopeptid in Helix- oder Faltblattstruktur enthalten, gekennzeichnet.

Membran-Biosensoren des oben bezeichneten prinzipiellen Aufbaus können u.a. zwei Aufgaben erfüllen:
- Liganden, die eine spezifische biologische Wechselwirkung mit dem eingebauten Rezeptor aufweisen, können als Analyten in Flüssigkeiten biologischen Ursprungs detektiert werden, woraus medizintechnische Teststäbchen oder andere medizinische oder biologische Analysenmethoden entwickelt werden können; und
- verschiedene Liganden in geeigneten Lösungsmitteln können auf ihre Eignung zur Paarbildung (spezifische Wechselwirkung) mit einem vorgegebenen Rezeptor überprüft werden, woraus beispielsweise die Entwicklung von chemisch-biologischen Bausteinen für neue Wirkstoffe auf dem Gebiet der Pharmazeutika und der Pflanzenschutzmittel erkannt und gezielt entwickelt werden können.

Rezeptoren können die genannten Wirkungen in biologischen Systemen nur entfalten, wenn sie sich innerhalb einer Zellmembran befinden. Solche Zellmembranen bestehen im allgemeinen aus einer Lipiddoppelschicht. Beispielsweise kann die Anbindung eines zum vorliegenden Rezeptor spezifischen Liganden eine Öffnung des Ionenkanals dieses Rezeptors bewirken, so daß ein elektrisches Signal durch die Lipiddoppelschicht in das Innere einer biologischen Zelle oder aus dem Inneren einer solchen biologischen Zelle heraus möglich ist. Künstliche Membran-Biosensoren, die für die obengenannten analytischen und sonstigen Zwecke einsetzbar sein sollen, müssen jedoch zur besseren Handhabbarkeit und Haltbarkeit durch einen Festkörper unterstützt sein. Eine darauf angebrachte Lipiddoppelschicht bedeutet sodann die Nachstellung der für die Einlagerung des Rezeptors notwendigen Zellmembran. Für die Sicherstellung der Wirksamkeit des Rezeptors, der in Zellen biologischen Ursprungs in das Zellinnere hineinreicht und dort nur Kontakt mit der Zellflüssigkeit hat, ist es notwendig, einen Kontakt des Rezeptors mit dem unterstützenden Festkörper zu vermeiden.

Zur Vermeidung der Berührung der unterstützenden Festkörper durch den Rezeptor wurde daher die Lipiddoppelschicht auf ihrer dem Träger zugewandten Seite mit einem Spacer aus Polvoxyethylen-(Polyoxyalkylen-)Gruppen versehen (EP 441 120-A; WO 93/21528; Langmuir 10 (1994), 197-210). Biosensoren der genannten Art scheinen jedoch bei ihrer Herstellung und ihrem praktischen Gebrauch schwierig zu sein, da die Polyoxyalkylen-Brückenglieder (Spacer) in Self-Assembly-(SA-)Verfahren keine ausreichend stabilen Ordnungszustande ergaben, wodurch Gebrauch und Reproduzierbarkeit der Ergebnisse stark eingeschränkt wurden.

Es wurde nunmehr gefunden, daß die genannten Nachteile überwunden werden können, wenn Oligopeptid enthaltende Spacer-Gruppen eingesetzt werden.

Die Erfindung betrifft durch Festkörper unterstützt Membran-Biosensoren, bestehend aus einem Festkörper A als Träger, eine Lipiddoppelschicht B als Membran mit zwischen A und B eingebauten Spacern C und einem in die Lipiddoppelschicht eingelagerten Rezeptor D, wobei
a) A auf seiner D zugewandten Oberfläche aus einem korrosionsfesten Material mit einem Abgriff eines elektrischen Signals besteht,
b) die untere Lipidmonoschicht von B zu 1-40 % aller Lipidmoleküle aus Di-(C₈-C₃₀-acyl)-phosphatidyl-Verbindungen mit einer natürlich vorkommenden Kopfgruppe und zu 60-99 % aus einem Di-(C₈-C₃₀-acyl)-phosphatid, das jedoch an Stelle der Kopfgruppe den Spacer C trägt, besteht und dessen obere Lipidmonoschicht zu 100 % aus Di-(C₈-C₃₀-acyl)-phosphatidyl-Verbindungen mit einer natürlich vorkommenden Kopfgruppe besteht, wobei alle Acylgruppen einer Schicht im wesentlichen gleich lang sind, jedoch die Acylgruppen der unteren Lipidmonoschicht gleich oder verschieden lang wie die der oberen Lipidmonoschicht sind,
c) C aus 1 Molekül Ethanolamin, das eine Esterbindung zur Phosphatgruppe von B ausbildet, einem Oligopeptid in Helix- oder Faltblattstruktur aus 4-20 C₂-C₁₀-α-Aminosäuren und einer Ankergruppe, die eine chemische oder physikochemische Bindung mit A eingeht, besteht, wobei alle C des Biosensors gleich sind, und
d) D keinen Berührungskontakt mit A hat.

Die Erfindung betrifft ferner ein Verfahren zur Herstellung eines durch Festkörper unterstützten Membran-Biosensors, wie oben beschrieben, das dadurch gekennzeichnet ist, daß man entweder
(1) eine Lösung von 1 Teil mit einem Spacer versehenen Lipids und 1-10⁶ Teilen nicht gespacerten Lipids in Wasser, einem organischen Lösungsmittel aus der Gruppe der C₁-C₄-Alkanole und C₃-C₆-Ketone oder einer Mischung mehrerer von ihnen auf einen Träger A bringt, wobei sich spontan eine untere Lipidmonoschicht, bestehend aus 60-99 % mit dem Spacer versehenen Lipidmolekülen und aus 1-40 % nicht gespacerten Lipidmolekülen, ausbildet, wobei sich
   (1a) aus wäßrigen Lösungen spontan zusätzlich auch die obere Lipidmonoschicht ausbildet, oder
   (1b) bei aus nicht Wasser enthaltenden Lösungen sich nur die untere Lipidmonoschicht ausbildet, die nach einem Spülvorgang mit dem verwendeten organischen Lösungsmittel durch Aufbringen einer Lösung von nicht gespacerten Lipidmolekülen in Wasser zur Lipiddoppelschicht vervollständigt wird,
   und eine Micellösung einer Rezeptorsuspension auf die nach (1a) oder (1b) gebildete Lipiddoppelschicht bringt, wobei sich der Rezeptor D spontan in die Lipiddoppelschicht einordnet, oder
(2) eine Lösung von mit einem Spacer versehenen Lipid in einem der obigen Lösungsmittel auf einen Träger A bringt, nach Ausbildung einer Submonolage des Lipids diese Lösung wieder vom Träger entfernt, die auf dem Träger gebildete Submonolage mit einem der obigen Lösungsmittel spült und dann eine micellare Lösung aus Wasser, nicht gespacertem Lipid, Tensid und Rezeptor D auf die Submonolage bringt, wobei sich die Lipiddoppelschicht spontan vervollständigt und sich der Rezeptor D spontan in die Lipiddoppelschicht einordnet, oder
(3) auf die auf dem Träger A gebildete Submonolage eine Vesikellösung, bestehend aus Rezeptormolekülen und Lipiden, aufbringt, wobei die Vesikel spontan auf der Submonolage fusionieren und sich der Rezeptor D spontan in die Lipiddoppelschicht einordnet.

Als Träger A kommen beliebige dimensionsstabile Festkörper in Frage, die aus einem keramischen Stoff, einem Metall oder einem duroplastischen oder thermoplastischen Polymer bestehen können, der auf seiner dem Rezeptor D zugewandten Oberfläche aus einem korrosionsfesten Material mit einem Abgriff eines elektrischen Signals besteht. Für den Fall, daß der Träger aus einem korrosionsfesten metallischen Werkstoff besteht, braucht er an seiner Oberfläche nicht weiter verändert oder beschichtet zu werden. Dies trifft zu beispielsweise für (Halb)Edelmetalle wie Gold, Silber, Platin, Palladium, Rhodium, Ruthenium, Iridium oder Kupfer. Besteht der Träger aus einem nicht korrosionsfesten Metall, muß er auf seiner D zugewandten Oberfläche mit einem der genannten korrosionsfesten (Halb)Edelmetalle plattiert oder anderweitig (z.B. naßchemisch oder elektrolytisch) beschichtet werden.

Ein korrosionsfestes Trägermaterial, das zugleich den Abgriff eines elektrischen Signals ermöglicht, ist weiterhin dotiertes Silizium.

Bei nicht korrosionsfesten metallischen Werkstoffen oder bei duroplastischen oder thermoplastischen Polymeren muß die Oberfläche, die dem Rezeptor D zugewandt ist, durch eines der genannten korrosionsfesten (Halb)Edelmetalle plattiert oder anderweitig beschichtet werden.

Auf dem Träger A wird eine Lipiddoppelschicht B angeordnet. Diese Lipiddoppelschicht besteht aus einer Vielzahl von Molekülen von Di-(C₈-C₃₀-acyl)-phosphatidyl-cholin, die mit den Kohlenwasserstoffenden der Acylgruppen zueinander gerichtet sind und eine hoch ausgerichtete Struktur aufweisen. Während die obere Schicht dieser Doppelschicht zu 100 % aus solchen Phosphatidyl-cholin-Molekülen besteht, ist die untere Schicht der Doppelschicht B nur zu 1-40 % aller Lipidmoleküle aus Di-(C₈-C₃₀-acyl)-phosphatidyl-cholin aufgebaut und zu 60-99 % aus einem Di-(C₈-C₃₀-acyl)-phosphatid, bei dem an Stelle des Cholins ein Spacer C tritt. In bevorzugter Weise bestehen die erfindungsgemäßen Biosensoren in der unteren Lipidmonoschicht von B zu 10-30 % aller Lipidmoleküle aus Di-(C₈-C₃₀-acyl)-phosphatidyl-cholin und zu 70-90 % aus einem Di-(C₈-C₃₀-acyl)-phosphatid, das jedoch anstelle des Cholins den Spacer C trägt, und in der oberen Schicht zu 100 % aus Di-(C₈-C₃₀-acyl)-phosphatidyl-cholin. In weiterhin bevorzugter Weise enthalten die Acylgruppen 10-24 C-Atome.

Die beiden langkettigen Fettsäuren, deren Acylgruppen mit dem Glycerin vestert sind, haben in Form der Acylgruppen 8-30 C-Atome, bevorzugt 10-24 C-Atome. Ein typischer Acylrest ist der Myristylrest -CO-(CH₂)₁₂-CH₃ mit insgesamt 14 C-Atomen. Andere Beispiele für Acylgruppen, die erfindungsgemäß geeignet sind, sind der Caprylylrest -CO-(CH₂)₆-CH₃ mit 8 C-Atomen, der Capronylrest -CO-(CH₂)₈-CH₃ mit 10 C-Atomen, der Laurylrest, der Palmitylrest, der Stearylrest, der von der n-Eicosancarbonsäure abgeleitete Arachinylrest (C₂₀), der von der Tetracosan-carbonsäure abgeleitete Lignocerylrest (C₂₄) und der von der Triacontancarbonsäure abgeleitete Acylrest (C₃₀). Alle in Frage kommenden Acylreste sind geradkettig. Außer den bereits aufgeführten Acylresten mit geradzahliger C-Atomzahl sind auch die mit ungeradzahliger C-Atomzahl einsetzbar, beispielsweise der Acylrest der Tridecan-carbonsäure (C₁₃), der Acylrest der Pentadecan-carbonsäure (C₁₅), der Acylrest der Heptadecancarbonsäure (Margarinsäure; C₁₇), der Acylrest der Heneicosan-säure (C₂₁) und andere. Außer den gesättigten Acylresten mit geradzahliger oder ungeradzahliger C-Atomzahl kommen ungesättigte Acylreste in Frage, wie Oleyl (C₁₈), Elaidinyl (C₁₈), Linolyl (C₁₈), Linolenyl (C₁₈) oder ihre Homologen mit abweichender C-Atomzahl. Lediglich aus Gründen der besseren Verfügbarkeit bevorzugt man Acylreste mit geradzahligen C-Atomzahlen.

In der Lipiddoppelschicht B des erfindungsgemäßen Membran-Biosensors stehen sich die beiden Lipidmonoschichten mit den Kohlenwasserstoffresten der Acylgruppen gegenüber.

Innerhalb jeder Lipidmonoschicht haben alle darin vorhandenen Acylgruppen im wesentlichen die gleiche Kettenlänge. Dies bedeutet, daß in Acylgruppen einer vorgesehenen Kettenlänge innerhalb des oben genannten Bereichs für eine Lipidmonoschicht auch bis zu 30 Mol-% solche der anschließenden Kettenlängen mit bis zu 2 C-Atomen weniger bzw. mehr vorhanden sein können. Bei einer vorgesehenen Kettenlänge von 16 C-Atomen können also bis zu insgesamt 30 Mol-% solche mit 14, 15, 17 bzw. 18 C-Atomen oder Gemische hiervon vorliegen. Aus Gründen der vereinfachten Handhabung kann es sinnvoll und vorteilhaft sein, in beiden Lipidmonoschichten Acylgruppen gleicher Kettenlänge einzubauen. Dies ist auch in den oben beschriebenen Herstellungsverfahren zur Ausbildung des erfindungsgemäßen Membran-Biosensors gemaß (1a) und (2), bei denen sich die Lipiddoppelschicht selbst organisiert, notwendig, um im wesentlichen gleiche Kettenlängen in den jeweiligen Lipidmonoschichten sicherzustellen. Die Herstellungsvariante (1b), bei der sich gezielt zunächst nur die untere Lipidmonoschicht herstellen laßt, ermöglicht es jedoch, die separat aufgetragene obere Lipidmonoschicht mit Acylgruppen, die eine gegenüber der unteren Lipidmonoschicht verschiedene Zahl von C-Atomzahlen hat, aufzutragen. Innerhalb der aufgetragenen oberen Lipidmonoschicht haben jedoch wiederum alle Acylgruppen die gleiche Kettenlänge.

Der Spacer C besteht aus einem Molekül Ethanolamin, das esterartig an die Phosphatgruppe des Diacylphosphatids gebunden ist. An das N-Atom des Ethanolamins ist sodann ein Oligopeptid aus 4-20 α-Aminosäuren gebunden. In bevorzugter Weise handelt es sich um ein Oligopeptid aus 4-10 α-Aminosäuren. Die α-Aminosäuren enthalten 2-10 C-Atome, bevorzugt 2-4 C-Atome, besonders bevorzugt handelt es sich um Glycin mit 2 C-Atomen. Oligopeptid-Spacer eines solchen Aufbaus liegen in einer Helix- oder Faltblattstruktur vor und ermöglichen dadurch eine hohe Ordnung im Verhältnis zwischen der unteren Monoschicht der Lipiddoppelschicht und dem Träger A. Diese hohe Ordnung ermöglicht einerseits eine der natürlichen Zellmembran nachempfundene Umgebung für den Rezeptor D und vermeidet andererseits einen Kontakt dieses Rezeptors D mit dem Träger A. Die Variabilität dieses Spacers im erfindungsgemäßen Membran-Biosensor erlaubt es, auf die Größe und den Raumbedarf des Rezeptors D gezielt einzugehen. Der Spacer trägt schließlich mit Bindung an das N-Atom der terminalen α-Aminosäure eine Ankergruppe, die es erlaubt, die gespacerte untere Lipidmonoschicht der Lipiddoppelschicht B auf der Oberfläche des korrosionsfesten Materials mit dem Träger A, der den Abgriff eines elektrischen Signals erlaubt, zu fixieren. Wie oben bereits dargestellt wurde, ist die korrosionsfeste Oberfläche des Trägers A, die den Abgriff eines elektrischen Signals erlaubt, ein (Halb)Edelmetall, so daß als Ankergruppe eine solche in Frage kommt, die beispielsweise eine Thiol- oder Disulfidgruppe hat. Eine Thiol- oder Disulfidgruppe kommt insbesondere bei einem der (Halb)Edelmetalle, besonders bei Gold oder bei Platin in Frage. Für den Fall, daß die korrosionsfeste und den Abgriff eines elektrischen Signals erlaubende Oberfläche beispielsweise dotiertes Silizium ist, kommt als Ankergruppe auch eine mit einer Silan-Gruppierung in Frage.

Solche Thiol-, Disulfid- oder Silan-Gruppen stellen eine chemische oder physikochemische Bindung mit dem Träger A her. Weitere Ankergruppen sind: Carboxylgruppen, Isocyanate, Säureanhydride. Der Aufbau der erfindungsgemäß vorhandenen beiden Lipidschichten der Lipiddoppelschicht B ist den natürlich vorkommenden Phosphatiden, Sphingomyelinen und Cholesterol in Zellmembranen nachgestellt. Phosphatide, die natürlich vorkommen, sind solche, bei denen an das Glycerin zwei langkettige Fettsäuren und ein Phosphorsäurerest gebunden sind (Phosphatidsäure), wobei an den Phosphorsäurerest eine weitere Verbindung aus der Gruppe der natürlich vorkommenden Kopfgruppen von Phospholipiden gebunden ist; solche Kopfgruppen sind beispielsweise: Cholin, Glycerin, Ethanolamin: Serin und Inositol. In allen genannten Fällen stellen diese Verbindungen Amphiphile dar; bevorzugt sind solche, die die Aktivität des Rezeptors erhalten. In den nicht gespacerten Lipidschichten ist diese Base in bevorzugter Weise das bereits genannte Cholin. Weitere Basen außer Cholin sind die oben genannten. In den mit einem Spacer ausgerüsteten Lipideinheiten tritt in der oben beschriebenen Weise an die Stelle des Cholins der Spacer mit dem Ethanolamin und dem sich daran anschließenden weiteren, oben beschriebenen Aufbau des gesamten Spacers einschließlich der Ankergruppe.

Damit stehen nunmehr auf dem Träger A fixierte Lipiddoppelschichten B zur Verfügung, die mit Hilfe der in die untere Lipidmonoschicht eingebauten Spacer C einen vorausbestimmbaren Abstand zum Träger A haben. Diese Lipiddoppelschichten simulieren eine natürliche Zellmembran. In diese Lipiddoppelschichten kann nunmehr ein Rezeptor D eingebracht werden. Solche Rezeptoren D haben einen hydrophoben membrandurchspannenden Teil mit hydrophilen Domänen unterschiedlicher Größe. Solche Rezeptoren können außerdem unterschiedliche Längen aufweisen. Das Einbringen dieser Rezeptoren D in die Lipiddoppelschicht B erfolgt spontan durch Selbstorganisation von D in B. Durch den Aufbau der Lipiddoppelschicht B mit unterschiedlich langen Acylresten und mit unterschiedlich langen Spacergruppen C kann auf den Raumbedarf unterschiedlich langer Rezeptoren D Rücksicht genommen werden, ohne daß solche Rezeptoren D Kontakt mit dem Träger A erhalten. Durch die im Herstellungsverfahren (2) zunächst aufgetragene Submonolage kann weiterhin der gezielte Prozentsatz an unterer Lipidmonoschicht eingestellt werden, die einen Spacer und eine Ankergruppe trägt.

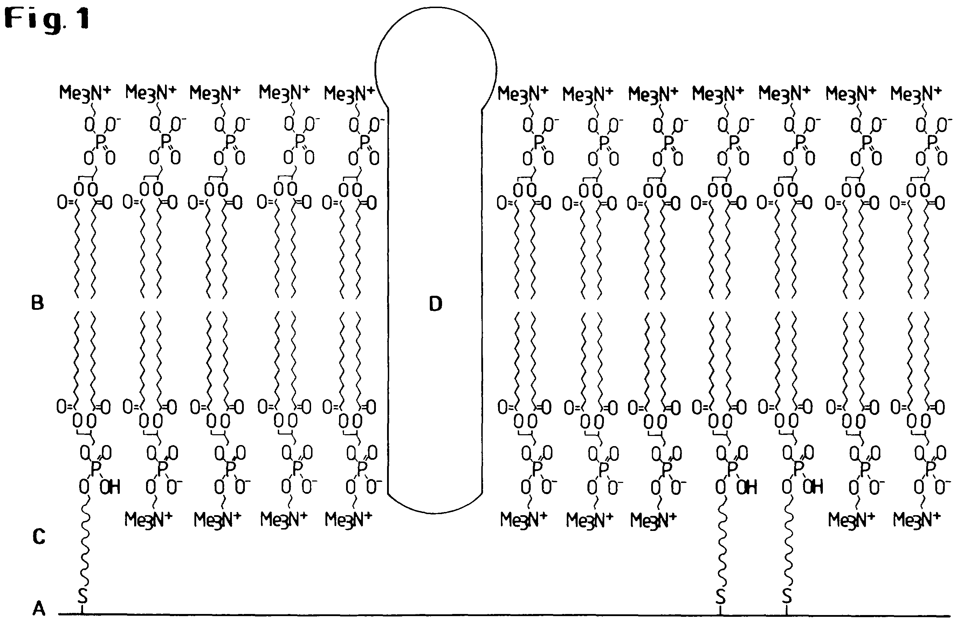

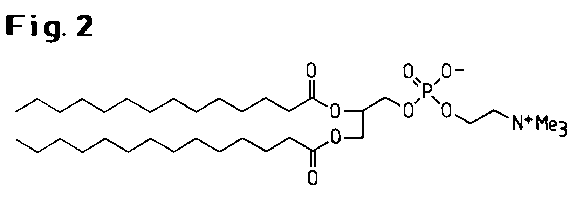

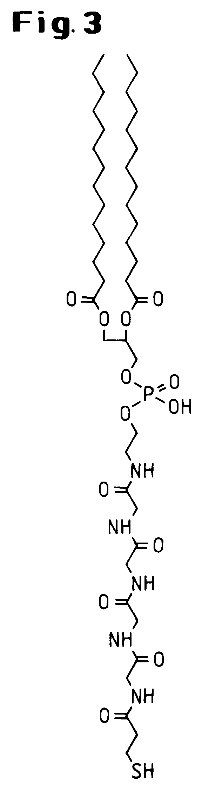

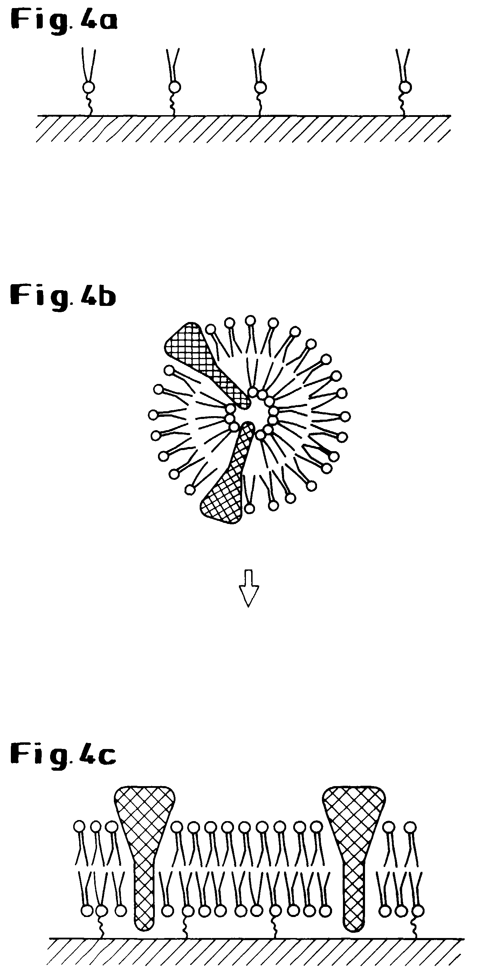

Die beigefügten Fig. 1, 2, 3 und 4a bis 4c verdeutlichen den Aufbau und eine Herstellungsvariante der erfindungsgemäßen Membran-Biosensoren. In Fig. 1 bedeutet A die Oberfläche des als Träger dienenden Festkörpers, die aus einem korrosionsfesten Material mit einem Abgriff eines elektrischen Signals besteht, B die Lipiddoppelschicht, in der beide Lipidmonoschichten mit ihren langkettigen Kohlenwasserstoffresten der Acylgruppen zueinander stehen, C in stilisierter Weise den Spacer, mit dem einzelne Lipidmoleküle der unteren Lipidmonoschicht an die Oberfläche des Trägers A gebunden sind, und D einen eingelagerten Rezeptor. In Fig. 2 wird der Aufbau nicht gespacerter Lipidmoleküle gezeigt, die von oben nach unten angeordnet folgende Bestandteile aufweisen: zwei gleich lange Acylreste (in Fig. 1, Fig. 2 und der noch folgenden Fig. 3 jeweils am Beispiel des Myristylrestes (C₁₄) demonstriert), den Glycerinrest, den Phosphorsäurerest und den Cholinrest. Fig. 3 zeigt den Aufbau eines mit einem Spacer C versehenes Lipidmolekül mit den von oben nach unten wie folgt angeordneten Resten: zwei gleich lange Acylreste, den Glycerinrest, den Phosphorsäurerest, den Ethanolaminrest, vier Glycinreste, die den Oligopeptid-Spacerteil verkörpern und das Mercaptopropionsäureamid als Ankergruppe. Fig. 4a bis 4c zeigt schließlich den schrittweisen Aufbau der erfindungsgemäßen Membran-Biosensoren im Sinne der oben beschriebenen Herstellungsvariante (3). Fig. 4a zeigt hierbei das Aufbringen von mit Spacern C einschließlich Ankergruppen versehenen Lipidmolekülen (gewellte Linie stellt den gesamten Spacer C dar, der innen weiße Kreis stellt den Phosphorsäure-Glycerin-Teil des Lipids dar, die beiden vom innen weißen Kreis abgehenden Linien stellen die beiden Acylreste dar). Der von Fig. 4a nach Fig. 4b weisende Pfeil stellt den nächsten Schritt dar, nämlich die Zugabe einer versikulären Lösung, die aus nicht gespacerten Phospholipiden mit einem analogen Aufbau zu Fig. 4a, jedoch ohne Spacer C und Rezeptoren zusammengesetzt ist.

Der von Fig. 4b nach Fig. 4c zeigende Pfeil stellt sodann die spontane Selbstorganisation der weiter oben beschriebenen Lipiddoppelschicht mit eingelagerten Rezeptoren dar. Es ist erkennbar, daß die in Fig. 4a hergestellte Submonoschicht mit nicht gespacerten Lipidmolekülen aufgefüllt wird und daß die obere Lipidmonoschicht nur aus nicht gespacerten Lipidmolekülen besteht.

### Beispiele

1.1. Präparation der Goldsubstrate (Festkörper A als Träger)
   Das zu bedampfende Substrat, benutzt wurden unter anderem Glas oder Silizium, wurde in heißer 2 %iger Detergenzlösung (70°C) unter Ultraschall 15 min gereinigt und anschließend 20 min mit Reinstwasser gespült, um restliches Detergenz zu entfernen. Das Substrat wurde in einem Argonplasma von anhaftenden Adsorbaten befreit und in einer Aufdampfanlage mit einer dünnen Chromschicht von ca. 10-20 nm und anschließend mit einer Goldschicht von 100-200 nm bedampft. Der elektrische Abgriff erfolgte direkt an der Goldschicht.
1.2. Präparation der ersten Monoschicht
   Zur Präparation einer Lipidmonoschicht, bestehend aus einem Dimyristylphopshatidylethanolamin, welches einen Spacer aus fünf Glycinresten und eine endständige Thiolgruppe trug, wurde das Argon-Plasma-gereinigte Goldsubstrat mit einer 0,1-1 mM enthaltenden ethanolischen Lösung dieses Spacerlipids inkubiert. Der Self-Assembly-Prozeß wurde nach 1-5 min abgebrochen, so daß eine unvollständige Lipidmonoschicht mit einem Belegungsgrad von etwa 70 % erhalten wurde. Anschließend wurde das beschichtete Goldsubstrat intensiv mit Ethanol gespült.
1.3. Rekonstitution des Acetylcholinrezeptors in Vesikel
   Ausgehend von isolierten Membranfragmenten aus Torpedo marmorata wurde der nikotinische Acetylchlolinrezeptor in Vesikel mit Hilfe der Detergenz-Verdünnungsmethode rekonstituiert. Membranfragmente mit 1 nmol α-Bungarotoxin-Bindungsstellen, 5 mg 1-Palmityl-2-oleyl-phosphatidylcholin und 2 % Natriumcholat wurden in einem Tris-Puffer suspendiert und im Ultraschallbad bis zu einer klaren micellaren Lösung beschallt. Diese Lösung wurde 12 h gegen reinen Puffer dialysiert.
1.4. Präparation einer Lipiddoppelschicht (Lipiddoppelschicht B mit Spacer C) mit rekonstituiertem Acetylcholinrezeptor (eingelagerter Rezeptor D)
   Die aus 1.3 erhaltenen Vesikel mit rekonstituiertem Rezeptorprotein wurden zu der hydrophoben trägerfixierten Monoschicht in einem TrisPuffer gegeben und die Fusion der Vesikel mit der Oberfläche bei 30-40°C eingeleitet. Nach Beendigung des Fusionsprozesses, der in der Regel nach ein bis zwei Stunden abgeschlossen ist, wurde die Vesikellösung gegen reine Pufferlösung ausgetauscht.

## Patentansprüche

1. Durch Festkörper unterstützte Membran-Biosensoren, bestehend aus einem Festkörper A als Träger, einer Lipiddoppelschicht B als Membran mit zwischen A und B eingebauten Spacern C und einem in die Lipiddoppelschicht eingelagerten Rezeptor D, wobei
a) A auf seiner D zugewandten Oberfläche aus einem korrosionsfesten Material mit einem Abgriff eines elektrischen Signals besteht,
b) die untere Lipidmonoschicht von B zu 1-40 % aller Lipidmoleküle aus Di-(C₈-C₃₀-acyl)-phosphatidyl-Verbindungen mit einer natürlich vorkommenden Kopfgruppe und zu 60-99 % aus einem Di-(C₈-C₃₀-acyl)-phosphatid, das jedoch an Stelle der Kopfgruppe den Spacer C trägt, besteht und dessen obere Lipidmonoschicht zu 100 % aus Di-(C₈-C₃₀-acyl)-phosphatidyl-Verbindungen mit einer natürlich vorkommenden Kopfgruppe besteht, wobei alle Acylgruppen einer Schicht im wesentlichen gleich lang sind, jedoch die Acylgruppen der unteren Lipidmonoschicht gleich oder verschieden lang wie die der oberen Lipidmonoschicht sind,
c) C aus 1 Molekül Ethanolamin, das eine Esterbindung zur Phosphatgruppe von B ausbildet, einem Oligopeptid in Helix- oder Faltblattstruktur aus 4-20 C₂-C₁₀-α-Aminosäuren und einer Ankergruppe, die eine chemische oder physikochemische Bindung mit A eingeht, besteht, wobei alle C des Biosensors gleich sind, und
d) D keinen Berührungskontakt mit A hat.

2. Verfahren zur Herstellung eines durch Festkörper unterstützten Membran-Biosensors nach Anspruch 1, dadurch gekennzeichnet, daß man entweder
(1) eine Lösung von 1 Teil mit einem Spacer versehenen Lipid und 1-10⁶ Teilen nicht gespacerten Lipids in Wasser, einem organischen Lösungsmittel aus der Gruppe der C₁-C₄-Alkanole und C₃-C₆-Ketone oder einer Mischung mehrerer von ihnen auf einen Träger A bringt, wobei sich spontan eine untere Lipidmonoschicht, bestehend aus 60-99 % mit dem Spacer versehenen Lipidmolekülen und aus 1-40 % nicht gespacerten Lipidmolekülen, ausbildet, wobei sich
(1a) aus wasserhaltigen Lösungen spontan zusätzlich auch die obere Lipidmonoschicht ausbildet, oder
(1b) bei aus nicht Wasser enthaltenden Lösungen sich nur die untere Lipidmonoschicht ausbildet, die nach einem Spülvorgang mit dem verwendeten organischen Lösungsmittel durch Aufbringen einer Lösung von nicht gespacerten Lipidmolekülen in Wasser zur Lipiddoppelschicht vervollstandigt wird,
und eine Micellösung einer Rezeptorsuspension auf die nach (1a) oder (1b) gebildete Lipiddoppelschicht bringt, wobei sich der Rezeptor D spontan in die Lipiddoppelschicht einordnet, oder
(2) eine Lösung von mit einem Spacer versehenen Lipid in einem der obigen Lösungsmittel auf einen Träger A bringt, nach Ausbildung einer Submonolage des Lipids diese Lösung wieder vom Träger entfernt, die auf dem Träger gebildete Submonolage mit einem der obigen Lösungsmittel spült und dann eine micellare Lösung aus Wasser, nicht gespacertem Lipid, Tensid und Rezeptor D auf die Submonolage bringt, wobei sich die Lipiddoppelschicht spontan vervollständigt und sich der Rezeptor D spontan in die Lipiddoppelschicht einordnet, oder
(3) auf die auf dem Träger A gebildete Submonolage eine Versikellösung, bestehend aus Rezeptormolekülen und Lipiden, aufbringt, wobei die Versikel spontan auf der Submonolage fusionieren und sich der Rezeptor D spontan in die Lipiddoppelschicht einordnet.

3. Biosensor nach Anspruch 1, dadurch gekennzeichnet, daß die untere Lipidmonoschicht von B zu 10-30 % aller Lipidmoleküle aus Di-(C₈-C₃₀-acyl)-phosphatidyl-cholin und zu 70-90 % aus einem Di-(C₈-C₃₀-acyl)-phosphatid, das jedoch an Stelle des Cholins den Spacer C trägt, besteht.

4. Biosensor nach Anspruch 1, dadurch gekennzeichnet, daß die Acylgruppen 10-24 C-Atome enthalten.

5. Biosensor nach Anspruch 1, dadurch gekennzeichnet, daß als α-Aminosäuren für das Oligopeptid solche mit 2-4 C-Atomen, bevorzugt das Glycin, eingesetzt werden.

6. Biosensor nach Anspruch 1, dadurch gekennzeichnet, daß die D zugewandte Oberfläche von A aus Gold oder Platin besteht.

7. Biosensor nach Anspruch 6, dadurch gekennzeichnet, daß die Ankergruppe zum Eingehen einer chemischen Bindung mit der aus Gold oder Platin bestehenden Oberfläche von A eine Thiol- oder Disulfidgruppe enthält.
